# EUROPEAN PATENT APPLICATION

(11) **EP 4 763 271 A1**
(43) Date of publication of application: **24.06.2026**
(21) Application number: 24220682.9
(22) Date of filing: 17.12.2024
(51) Int. Cl.: A61N 1/05, A61N 1/36

(54) **SYSTEM FOR CONTROLLING SELECTIVE STIMULATION OF NEURAL TISSUE**

(71) Applicant: Stichting IMEC Nederland, 5656 AE Eindhoven (NL)
(72) Inventor: SCHNEPEL, Philipp, 5654 CS Eindhoven (NL); MIHAJLOVIC, Vojkan, 5643 AZ Eindhoven (NL); ROSSETTI, Nicolo, 5654 AN Eindhoven (NL)
(74) Representative: AWA Sweden AB

(57) **Abstract**

According to a first aspect of the present inventive concept there is provided a system for controlling selective stimulation of neural tissue of a subject, comprising an electrode arrangement and a stimulation signal generating unit configured to generate a first and second intermittent current waveforms. The stimulation signal generating unit is configured to output the first intermittent current waveform to a first pair of electrodes and the second intermittent current waveform to a second pair of electrodes, for causing interferential stimulation of a plurality of neurons in a first region and a second region of the neural tissue. The interferential stimulation of the neurons is configured to activate stimulation of the neurons in the first region at a first time point, and the neurons in the second region at a second time point. The stimulation signal generating unit is configured to control one parameter for controlling a relation between the first time point and the second time point. According to a second aspect, there is provided a method for controlling selective stimulation of neural tissue.

## Description

### Technical field

The present description relates to controlling selective stimulation of neural tissue of a subject. In particular, the present description relates to a system and a method for controlling selective stimulation of neural tissue of a subject, and a method for nerve stimulation for pain relief.

### Background

Neurostimulation may be used for controlling a function of an organ or part of a body of a living being, such as a human being, e.g., for alleviating or treating a number of conditions of the human being. Neurostimulation may involve a control of electrical signals transmitted through a nerve, so as to trigger or block signals transmitted by the nerve.

Neurostimulation may be used for pain management, helping to alleviate chronic pain conditions such as arthritis, fibromyalgia, and neuropathy by interrupting or blocking pain signals to the brain, effectively reducing the sensation of pain. Commonly, spinal cord stimulation (SCS) is used for managing chronic pain.

However, there are challenges associated with neurostimulation. In the case of SCS, one challenge lies in activating specific neurons in a particular temporal pattern. Standard SCS based on square pulses causes paresthesia, the feeling of tingling, numbness or "pins and needles", due to temporally precise, synchronous activation of neurons. Advanced methods such as continuous kHz- or burst-stimulation are paresthesia-free but lack accurate control of the temporal pattern of activation and may require high energy consumption of the stimulators (i.e., implantable pulse generators, IPGs).

Furthermore, all existing approaches have limited spatial precision and suffer from difficulties in selecting the optimal stimulation dosage due to movement artifacts and body posture changes when a patient is in movement compared to laying down.

Ensuring proper electrode placement and device settings can also be challenging, requiring professional guidance for optimal results.

There is a need for a spatially selective paresthesia-free neurostimulation.

### Summary

An objective of the present description is to provide accurate control of stimulation of neural tissue. A particular objective is to provide accurate control for spatially selective paresthesia-free neurostimulation.

This and other objectives are at least partly met by the invention as defined in the independent claims. Preferred embodiments are set out in the dependent claims.

According to a first aspect, there is provided a system for controlling selective stimulation of neural tissue of a subject, said system comprising an electrode arrangement, comprising a plurality of electrodes, wherein different electrodes of the plurality of electrodes are configured to be arranged in different electrode locations in relation to the neural tissue, and a stimulation signal generating unit, wherein the stimulation signal generating unit is configured to generate a first intermittent current waveform comprising a first pulse and a second intermittent current waveform comprising a second pulse, wherein the first pulse comprises a first frequency and the second pulse comprises a second frequency, wherein the second frequency is different from the first frequency, wherein the stimulation signal generating unit is configured to output the first intermittent current waveform to a first pair of electrodes of the electrode arrangement and the second intermittent current waveform to a second pair of electrodes of the electrode arrangement, for causing interferential stimulation of a plurality of neurons in at least a first region and a second region of the neural tissue, wherein the interferential stimulation of the neurons is configured to activate stimulation of the neurons in the first region at a first time point, and the neurons in the second region at a second time point, wherein the stimulation signal generating unit is configured to control at least one parameter of the first pulse or the second pulse, for controlling a relation between the first time point and the second time point.

The system may be used for controlling stimulation of neural tissue of a subject, wherein the subject may be a living being. It should be realized that the system may be used for controlling stimulation of a human being but that the system may likewise be used for controlling stimulation of neural tissue of an animal.

Neural tissue is found in the brain, spinal cord and nerves. The neural tissue may form part of the peripheral nervous tissue. It should be realized that, as used herein the term "neural tissue" may be used interchangeably with the term "nerve", "nervous tissue", "nervous system", "spinal cord" and "brain".

Neurons, also called nerve cells, form part of the neural tissue.

Neurons comprises axons and dendrites, however neurons commonly refer to only the cell bodies, which may be most prominent in gray matter while white matter mainly comprises axons, or nerve fibers. It is a realization that while this disclosure mainly references neurons, this may be interchangeably referenced as nerve fibers, depending on where in the subject the stimulation occurs. For example, when stimulating the vagus nerve, or white matter, it is most common to refer to the simulation as occurring in the axons, or nerve fibers, while when stimulation the spinal cord or other gray matter, it is most common to refer to the simulation as occurring in the cell bodies. The electrode arrangement comprises a plurality of electrodes and different electrodes of the plurality of electrodes are configured to be arranged in different electrode locations in relation to the neural tissue.

The neural tissue, such as a nerve, may have a longitudinal extension forming an elongate structure. The neural tissue may be configured to propagate signals along the longitudinal extension.

According to an embodiment, the electrodes within the first pair of electrodes of the electrode arrangement may be slightly displaced in relation to each other along the longitudinal extension of the neural tissue, and the electrodes within the second pair of electrodes of the electrode arrangement may be slightly displaced in relation to each other along the longitudinal extension of the neural tissue.

The electrodes of the first pair and the second pair of electrodes may be arranged at corresponding cross sections of the neural tissue, such that a first electrode of the first pair of electrodes and a first electrode of the second pair of electrodes are arranged in relation to a same first cross-section of the neural tissue, whereas a second electrode of the first pair of electrodes and a second electrode of the second pair of electrodes are arranged in relation to a same second cross section. It is a realization that, while the first electrode of the first pair of electrodes and the first electrode of the second pair of electrodes preferably are arranged in relation to a same first cross-section, this is not always the case due to movement artifacts caused by movement by the subject.

Thus, the system is adapted such that the plurality of electrodes may be arranged close to or in contact with the neural tissue to be stimulated. This implies that stimulation signals may be output by the system directly into the neural tissue providing an accurate control of the stimulation within the nerve.

The stimulation signal generating unit is configured to generate a first intermittent current waveform comprising a first pulse and a second intermittent current waveform comprising a second pulse.

The first pulse comprises a first frequency and the second pulse comprises a second frequency, wherein the second frequency is different from the first frequency.

The stimulation signal generating unit is configured to output the first intermittent current waveform to a first pair of electrodes of the electrode arrangement and the second intermittent current waveform to a second pair of electrodes of the electrode arrangement, thereby causing interferential stimulation of a plurality of neurons in at least a first region and a second region of the neural tissue.

The plurality of electrodes may comprise one or more pre-defined pairs of electrodes to be used for providing the interferential stimulation. However, according to an alternative, the electrodes forming a pair of electrodes used for providing the interferential stimulation may be dynamically selected among the plurality of electrodes.

According to an embodiment, the first pair of electrodes and the second pair of electrodes are arranged at different electrode locations in relation to a cross-section of the neural tissue. This may be understood as arranged at different electrode locations of a circumference of the neural tissue. Thus, the first intermittent current waveform and the second intermittent current waveform may mainly define electric fields based on the waveforms in different regions of the cross-section of the neural tissue. The first intermittent current waveform and the second intermittent current waveform may interfere within the neural tissue to stimulate the neural tissue through interferential stimulation.

It should be realized that both the first and second region of the neural tissue may be located in a common cross-section or part of the neural tissue. Thus, the first and the second regions may be arranged within a part of the neural tissue in which interferential stimulation is provided. Both electrodes of the first pair of electrodes may be arranged closer to the first region than the second region, while both electrodes of the second pair of electrodes may be arranged closer to the second region than the first region. However, it should be realized that the electrodes may be arranged in other relations to the first and second regions. For example, the first region may be arranged close to one of the pairs of electrodes and the second region may be arranged at a distance from a surface of the neural tissue and hence, farther away from the pairs of electrodes than the first region.

It is a realization that the system may be configured to cause interferential stimulations in more than two regions of the neural tissue. For example, a first region may be arranged close to the first pair of electrodes, a second region may be arranged close to the second pair of electrodes, a third region may be arranged at a distance from a surface of the neural tissue and hence, farther away from the pairs of electrodes than the first and the second region. A region may be defined as comprising at least one neuron.

The system is configured to provide a first intermittent current waveform and a second intermittent current waveform. Thus, a signal may be intermittently provided to the first pair of electrodes and a signal may be intermittently provided to a second pair of electrodes. The first waveform and the second waveform may each comprise a plurality of pulses. The first waveform and the second waveform need not be continuous over the plurality of pulses with the first waveform and/or the second waveform being modulated to form pulses of interferential stimulation with sufficient amplitude to activate the neurons of the neural tissue. Rather, the first pair of electrodes and the second pair of electrodes may only receive signals at time instances when stimulation of the neural tissue is to take place.

Thus, the first intermittent current waveform may provide a non-zero signal or high amplitude signal within the first pulse and may provide a zero-level signal between pulses within a sequence of first pulses. Also, the second intermittent current waveform may provide a non-zero signal or high amplitude signal within the second pulse and may provide a zero-level signal between pulses within a sequence of second pulses. It should be realized that instead of a zero-level signal, a low amplitude signal may be provided between the first pulses and between the second pulses, respectively. The low amplitude signal may have such a low amplitude that no stimulation of the neural tissue takes place through interferential stimulation and the amplitude may also be low as to not draw a lot of energy.

The first intermittent current waveform and the second intermittent current waveform may thus be called intermittent in that the waveforms intermittently have sufficiently high amplitude to cause interferential stimulation and therebetween have very low or zero amplitude to reduce power consumption. It should further be realized that the waveforms may comprise single pulses, respectively. Thus, each time a pulse is to be output, the stimulation signal generating unit may be controlled to generate a waveform comprising a single pulse and control of generation of the single pulse is not related to generation of other pulses.

Thus, thanks to the stimulation signal generated unit being configured to provide intermittent current waveforms, the stimulation of the neural tissue may be provided in an energy-efficient manner. The stimulation generating unit needs only output signals during the first pulses and the second pulses, respectively.

The stimulation generating unit is configured to generate current waveforms for providing a current to the neural tissue. The current may be converted to a voltage within the neural tissue based on transconductance of tissue such that an action potential for stimulating the neural tissue is formed.

The interferential stimulation is based on the difference in frequency of a first pulse in the sequence of first pulses and a second pulse in the sequence of second pulses. When the first intermittent current waveform and the second intermittent current waveform interfere, the interference may form a varying signal having a frequency corresponding to the difference in frequency of the first pulse and a second pulse. This frequency of the interferential signal may be referred to as a beat frequency.

The first frequency and the second frequency may be selected to be high frequencies, whereas the beat frequency represents a low frequency lower than the first frequency and the second frequency.

According to an embodiment, the stimulation generating unit is configured to generate a first frequency and a second frequency, respectively, being in a range of 100 Hz-1 MHz, such as 10 kHz-100 kHz.

Thus, the first stimulation generating unit and the second stimulation generating unit may generate signals having a relatively high frequency.

According to an embodiment, the stimulation signal generating unit is configured to generate the first intermittent current waveform and the second intermittent current waveform with a difference in frequency in a range of 1 Hz - 10 kHz, such as 1 - 5 kHz.

Thus, the beat frequency may be in the range of 1 Hz - 10 kHz or in the range of 1 - 5 kHz. The use of a beat frequency in these ranges may be suitable for providing stimulation of the neural tissue. The beat frequency may be selected in relation to a desired stimulation of the neural tissue, such as in relation to a particular stimulation paradigm to be used.

The interferential stimulation of the neurons is configured to activate stimulation of the neurons in the first region at a first time point, and the neurons in the second region at a second time point.

According to an embodiment, the first time point may be different from the second time point.

The interferential stimulation signal may be configured to provide an amplitude modulated signal, wherein an envelope curve of the signal may define the beat frequency. The interferential signal may include a sequence of cycles of increasing and decreasing amplitude within a single period of the envelope curve. Thus, a peak amplitude of respective cycles may be increasing in the sequence of cycles of the envelope curve. The interferential stimulation signal may be considered an amplitude modulated signal, with an amplitude that increases (or decreases) with time.

It is a realization that the envelope curve of the interferential stimulation signal will have a different amplitude modulation in different regions of the neural tissue. The interference between the first intermittent current waveform and the second intermittent current waveform will result in different interference patterns, in different regions of the neural tissue, due to an amplitude relation of the first intermittent current waveform and the second intermittent current waveform meeting in the respective region. The amplitude modulation may be larger towards a center of the neural tissue. Hence, neurons in central regions of the neural tissue may be activated later in time than neurons in more peripheral regions, closer to the electrodes, of the neural tissue.

Due to the difference in interferential pattern in different regions the interferential stimulation may activate the neurons in different regions at different time points.

By providing a stimulation signal comprising a fixed inter-pulse-interval (IPI) a synchronous and precise time-controlled pattern of neuron activation within the neural tissue may be provided. If the properties of the first and second intermittent current waveforms, the electrode locations and the inter-pulse interval is kept the same, the difference between the first time point, i.e., activation in the first region, and the second time point, i.e., activation in the second region, will be the same. Thus, by having the same inter-pulse-interval between each of the plurality of pulses of the first intermittent and each of the pulses of the plurality of second pulses, a repetitive activation pattern will we provided.

The timing of the first time point and the second time point may be controlled to provide a desired relation between activation of different regions of the neural tissue. This may be particularly useful for activation of neural signals for blocking the propagation of pain-related neural signals. The activation pattern may be controlled in order to avoid simultaneous stimulation of all neurons in the cross-section, which may cause paresthesia. Further, thanks to the control being able to provide a repetitive activation pattern, the stimulation of neurons in the first and second regions may be repetitively provided in a controlled manner without causing paresthesia. Thus, the device may be particularly useful for providing stimulation of neural tissue for pain relief while paresthesia may be avoided.

The stimulation signal generating unit is configured to control at least one parameter of the first pulse or the second pulse, for controlling a relation between the first time point and the second time point.

The stimulation signal generating unit may comprise a plurality of stimulation generating modules with separate current generators, each dedicated to generating the first waveform and the second waveform respectively. This implies that the first waveform to be output to the first pair of electrodes and the second waveform to be output to the second pair of electrodes may be separately controlled to ensure that desired waveforms are output and that a waveform is not affected by the generation of other waveforms in the stimulation signal generating unit. Hence, the interferential stimulation may be accurately controlled by separately controlling the first waveform and the second waveform.

Advantageously, the control provided can be used for ensuring that different neurons are stimulated according to a desired time pattern. For spinal cord stimulation, this should be used for controlling the relation between neurons such that they are activated at different time points to reduce paresthesia in case of pain treatment applications.

In the case of sensory afferent stimulation for sensory feedback targeting peripheral sensing nerves, the precise timing control of the activation can help mimic a pattern that would be provided by an expected natural sensory feedback.

Thanks to the interferential stimulation, the time point of activation for neurons within different regions may be controlled, thereby, producing spatially confined regions where the neurons experience delayed activation. The delay may be determined by the location of the stimulating electrodes in relation to the region, parameters of the first and second intermittent current waveforms and the interferential stimulation, and by properties inherent to the different neurons.

The pulses of the first and second intermittent current waveform allow for precise control of timing of activation of the neurons, and that may be further controlled by parameters of the pulse itself, such as stimulation amplitude, stimulation frequencies, the interferential beat frequency, and the duration of the beat as well as by the inter-pulse-interval (IPI).

The electrode arrangement may be adapted to provide the electrodes close to or in contact with the neural tissue to be stimulated. This implies that stimulation signals may be output by the electrode arrangement directly into the neural tissue providing an accurate control of the stimulation within the neural tissue. For example, the electrodes may be configured to be arranged at a surface of a nerve.

Stimulation of the neural tissue may correspond to providing a sufficiently large action potential as to trigger a signal to be transported by the neural tissue.

In an example, the system for controlling selective stimulation of neural tissue of a subject is used for pain relief.

According to an embodiment, the system further comprises a sensor arrangement configured to detect at least one of a neural activity or a physiological activity of the subject, wherein the sensor arrangement is configured to detect indications of changes in at least one of the neural activity or the physiological activity in response to the interferential stimulation of the neurons, and wherein the stimulation signal generating unit is configured to control the at least one parameter of the first pulse or the second pulse, based on the indications of changes in at least one of the neural activity or the physiological activity.

The sensor arrangement may comprise at least one sensor, configured to detect at least one of a neural activity or a physiological activity of the subject. The sensor is further configured to detect indications of changes in at least one the neural activity or the physiological activity in response to the interferential stimulation of the neurons by the electrodes. The sensor of the sensor arrangement may be configured to detect indications of change in a single neural activity or a single physiological activity, or may be configured to detect indications of change in more than one neural activity and/or physiological activity.

The sensor arrangement may comprise a plurality of sensors, wherein each of the sensors of plurality of sensors may be configured to detect at least one of a neural activity or a physiological activity.

It is a realization that the sensor arrangement may comprise a plurality of separate physical units.

Physiological activity may for example be heart rate, respiratory activity, gastrointestinal activity. Neural activity may for example be brain activity or PNS activity.

The sensor arrangement may be configured to at least partly be arranged within the subject. Alternatively, the sensor arrangement may be external to the subject.

The sensor arrangement may be a portable sensor arrangement. The sensor arrangement may be hospital equipment for measuring and/or monitoring different neural activities or physiological activities of the subject.

The stimulation signal generating unit may be configured to control the at least one parameter of the first pulse or the second pulse, based on the indications of changes in at least one of the neural activity or the physiological activity. Thus, the stimulation signal generating unit may adjust the interferential stimulation of the plurality of neurons in at least the first region and the second region of the neural tissue and by proxy controlling a relation between the first time point and the second time point, based on direct feedback from the sensor arrangement.

For example, when the system is used for controlling selective stimulation for pain relief, the at least one sensor may be used to detect a neural or physiological activity that may be directly related to pain experience, such as blood pressure or heart rate. The system may alternatively be configured to detect propagation of neural signals in the neural tissue to determine that desired stimulation of neural signals is achieved which may act to block pain signals from being propagated in the spinal cord. According to another example, the neural or physiological activity may be an unwanted side-effect of the stimulation. During optimal stimulation there may not be any changes in the neural or physiological activities, however due to movement of the subject the electrodes may shift location, and thus cause a new first and second region of the neural tissue to form. The stimulation signal generating unit may receive the detected indications of changes and adjust the at least one parameter of the first pulse or the second pulse based on the received input.

The stimulation signal generating unit may adjust one parameter of one of the first pulse and/or of the second pulse. As an alternative the stimulation signal generating unit may adjust a plurality of parameters of the first pulse and/or the second pulse.

The system allows controlling selective stimulation of neural tissue of a subject based on feedback on indications of changes in at least one of the neural activity or the physiological activity in response to the interferential stimulation from sensor arrangement. The system thus controls selective stimulation based on different physiological responses to different stimulation signals. The detected indications of changes in neural activity or physiological activity for the different responses provides input for controlling the selective stimulation.

Thanks to the system being configured to control selective stimulation of the neural tissue, based on feedback from the system itself, a control loop is achieved. Hence, the system advantageously may respond to changes in responses directly, thus achieving a high level of adaptability and accuracy, while minimizing unwanted responses.

Advantageously, the system may adapt itself to movement artifacts and body posture changes causing the electrode locations to shift.

According to an embodiment, the system further comprises a control unit configured to determine settings of the stimulation signal generating unit.

The control unit may be configured to determine settings of the stimulation signal generating unit comprising at least one of: stimulation amplitudes and stimulation frequencies of the first and second intermittent current waveform, the interferential beat frequency, and the duration of the beat as well as the inter-pulse-interval (IPI).

The control unit may be configured to store a pattern representing a desired time relation between the first time point and the second time point. The pattern representing a desired time relation may for example be a time difference between an indication of change of a first neural or physiological activity and an indication of change of a second neural or physiological activity. It is a realization that the pattern representing a desired time relation may be different depending on the stimulation location of the neural tissue, and the type of sensor arrangement used.

The control unit may comprise a data storage, wherein the data storage may be configured to store the pattern representing a desired time relation between the first time point and the second time point. In this example, the control unit may be configured to retrieve the stored patterns from the data storage.

The control unit may be further configured to receive feedback from the sensor arrangement representing an actual time relation between nerve signals relating to neurons in the first region and neurons in the second region, compare the received feedback to the stored pattern for determining a necessary change in the settings of the stimulation signal generating unit, and send a control signal to the stimulation signal generating unit for causing the stimulation signal generating unit to change that at least one parameter of the first pulse or the second pulse.

The control unit may be implemented in one or more general-purpose processing units, such as a central processing unit (CPU), which may execute instructions of one or more computer programs in order to implement functionality of the control unit. However, the control unit may alternatively be implemented as firmware arranged e.g., in an embedded system, or as a specifically designed processing unit, such as an Application-Specific Integrated Circuit (ASIC) or a Field-Programmable Gate Array (FPGA).

According to an embodiment, the at least one parameter of the first pulse or the second pulse, comprises at least one of an amplitude, a frequency, a starting time, a starting phase, or a pulse length.

By controlling the amplitude of the first intermittent current waveform and/or the second intermittent current waveform the spatial position of the interferential stimulation focal point may be possible to steer, thereby controlling a relation between the first time point and the second time point.

By controlling the pulse length, the first time point and/or the second time point, and the relation between the first time point and the second time point may be controlled. For example, a longer beat duration (pulse length) may increase the fiber activation delay, while a shorter beat duration may decrease the fiber activation delay, therefore enabling precise control of difference between the first and the second time point.

It is a realization that a longer beat durations may cause the stimulation signal to be ramped-up more slowly and over a longer period of time. Thanks to the charge being built-up more slowly, an increased activation delay compared to a shorter beat duration is achieved.

By controlling the at least one parameter a greater flexibility is provided. Furthermore, by controlling the at least one parameter based on direct feedback from the system itself an adaptable system is provided. The direct feedback allows for compensation for electrode displacements that may occur due to body posture changes. Furthermore, the system provides a greater flexibility.

According to an embodiment, the first intermittent current waveform comprises a first sequence of pulses and the second intermittent current waveform comprises a second sequence of pulses.

The stimulation signal generating unit may be configured to control an amplitude of a pulse of the first sequence following a preceding pulse of the first sequence, and control an amplitude of a pulse of the second sequence following a preceding pulse of the second sequence.

In other words, the first intermittent current waveform may comprise a plurality of subsequent pulses, also referred to as a first sequence of pulses. The first sequence of pulses may comprise at least two consecutive pulses, and wherein the time between the first pulse of the fist sequence of pulses and the second pulse of the first sequence of pulses may be referred to as the inter-pulse-interval.

The second intermittent current waveform may comprise a plurality of subsequent pulses, also referred to as a second sequence of pulses. The second sequence of pulses may comprise at least two consecutive pulses, and wherein the time between the first pulse of the second sequence of pulses and the second pulse of the second sequence of pulses may be referred to as the inter-pulse-interval.

The inter-pulse-interval for of first sequence of pulses and the inter-pulse-interval of the second sequence of pulses may be the same. Alternatively, the inter-pulse-interval for of first sequence of pulses and the inter-pulse-interval of the second sequence of pulses may be different.

The stimulation signal generating unit may be configured to change an amplitude of a pulse of the first sequence following a preceding pulse of the first sequence. Hence, the stimulation signal generating unit may be configured to change at least one property of a pulse of the first sequence of pulses of the first intermittent current waveform amidst the first sequence. The change may be triggered by a detected indication of change in a neural or physiological activity.

Similarly, the stimulation signal generating unit may be configured to change an amplitude of a pulse of the second sequence following a preceding pulse of the second sequence. The stimulation signal generating unit may be configured to change at least one property of a pulse of the first sequence of pulses of the first intermittent current waveform amidst the first sequence. The change may be triggered by a detected indication of change in a neural or physiological activity.

Thanks to the stimulating signal generating unit being configured to control an amplitude of a pulse amidst a sequence of pulses further improves the adaptability of the system. By allowing changes in amplitude of the pulses within a sequence of pulses a substantially immediate feedback-loop may be achieved.

According to an embodiment, the simulation signal generating unit is further configured to control the inter-pulse-interval in the first sequence of pulses and the second sequence of pulses.

The stimulation signal generating unit may be configured to control the inter-pulse-interval between preceding pulse and a pulse of the first sequence following a preceding pulse of the first sequence. It is a realization that the inter-pulse-interval may be the same between all subsequent pulses in a sequence of pulses. Alternatively, the inter-pulse-interval may be different between all subsequent pulses in a sequence of pulses.

According to an embodiment, the first region and the second region are different spatial regions in a cross-section of the neural tissue.

According to an embodiment, at least one of an envelope maximum or an envelope minimum of the interferential stimulation is different in the first region and in the second region. Hence, the time point at which stimulation of a neuron is activated is different for the different regions.

According to an embodiment, the stimulation signal generating unit comprises a first current generator, configured to generate the first intermittent current waveform, and a second current generator, configured to generate the second intermittent current waveform.

The system may thus comprise separate current generators for generating each of the first and second intermittent current waveforms respectively. This implies that generation of the first waveform to be output to the first pair of electrodes and generation of the second waveform to be output to the second pair of electrodes may be separately controlled. In addition, control of the inter-pulse-interval and control of an amplitude of a pulse in a sequence of pulses may be separately controlled for the first sequence of pulses of the first intermittent current waveform and the second sequence of pulses of the second intermittent current waveform respectively.

Hence, the stimulation of the neural tissue may be accurately controlled by separately controlling the first intermittent current waveform and the second intermittent current waveform.

According to an embodiment, the system is configured to be at least partially implanted at a spine of the subject, and wherein the neural tissue is a spinal cord of the subject.

This facilitates use of the system for providing stimulation of the neural tissue to the living being in a convenient way. In particular, when the system is implanted within the body of a human being, the stimulation of the neural tissue may be provided with minimal effect of everyday life of the human being.

However, the implanted system may still be configured to communicate with an external device through wireless communication. The system may receive control signals from the external device for changing parameters of the stimulation, such as parameters defining the stimulation paradigm, such as to control a location within the neural tissue to be stimulated or a frequency, an amplitude, or a duration of a stimulation to be provided. The external device may be provided in a housing that may be worn by the living being or may be adapted for being arranged in vicinity of the living being. The implanted system may be configured to communicate with a communication unit, which may be provided in a housing worn by the living being or arranged in vicinity of the living being wherein the communication unit may act as an intermediate device for allowing the implanted system to communicate with an external unit which may be remotely arranged. Thus, communication between the communication unit and the external device may be provided via a telecommunication or computer network.

According to an alternative embodiment, the system may be configured to be at least partially implanted in the subject.

According to a second aspect, there is provided a method for controlling selective nerve stimulation of neural tissue of a subject, said method comprising the steps of providing a first intermittent current waveform comprising a first pulse to a first pair of electrodes of an electrode arrangement, providing a second intermittent current waveform comprising a second pulse to a second pair of electrodes of the electrode arrangement, stimulating a plurality of neurons in at least a first region and a second region of the neural tissue using interferential stimulation based on interference between the first intermittent current waveform and the second intermittent current waveform, wherein the interferential stimulation of the neurons is configured to activate stimulation of the neurons in the first region at a first time point, and neurons in the second region at a second time point, and controlling a relation between the first time point and the second time point by the stimulation signal generating unit controlling at least one parameter of the first pulse or the second pulse.

This second aspect may generally present the same or corresponding advantages as the former aspect. Effects and features of this second aspect are largely analogous to those described above in connection with the first aspect. Embodiments mentioned in relation to the second aspect are largely compatible with the first aspect.

Thanks to the method, accurate control of selective nerve stimulation may be provided.

The aforementioned method may be implemented by a system comprising an electrode arrangement and a signal generating unit. For example, the method may be implemented using a system such as the system according to a first aspect of this disclosure.

According to a third aspect, there is provided a device for controlling selective nerve stimulation of neural tissue of a subject, said device being configured to control timing of generation of a first intermittent current waveform comprising a first pulse to be output to a first pair of electrodes of an electrode arrangement, control timing of generation of a second intermittent current waveform comprising a second pulse to be output to a second pair of electrodes of the electrode arrangement, wherein timing of the first and second intermittent current waveform controls timing of stimulation of a plurality of neurons in at least a first region and a second region of the neural tissue using interferential stimulation based on interference between the first intermittent current waveform and the second intermittent current waveform, and wherein the interferential stimulation of each of the neurons of the plurality of neurons in a first region is configured to activate stimulation of the neuron at a first time point, and the interferential stimulation of each of the neurons of the plurality of neurons in a second region is configured to activate stimulation of the neuron at a second time point, and wherein the device is further configured to control at least one parameter of the first pulse or the second pulse, for controlling a relation between the first time point and the second time point.

This third aspect may generally present the same or corresponding advantages as the former aspects. Effects and features of this third aspect are largely analogous to those described above in connection with the first and second aspects. Embodiments mentioned in relation to the third aspect are largely compatible with the first and second aspects.

Thanks to the device, accurate control of selective nerve stimulation may be provided.

According to a fourth aspect, there is provided a computer implemented method for controlling selective nerve stimulation of neural tissue of a subject, said method comprising controlling timing of generation of a first intermittent current waveform comprising a first pulse to be output to a first pair of electrodes of an electrode arrangement, controlling timing of generation of a second intermittent current waveform comprising a second pulse to be output to a second pair of electrodes of the electrode arrangement, wherein timing of the first and second intermittent current waveform controls timing of stimulation of a plurality of neurons in at least a first region and a second region of the neural tissue using interferential stimulation based on interference between the first intermittent current waveform and the second intermittent current waveform, wherein the interferential stimulation of each of the neurons of the plurality of neurons in a first region is configured to activate stimulation of the neuron at a first time point, and the interferential stimulation of each of the neurons of the plurality of neurons in a second region is configured to activate stimulation of the neuron at a second time point, and controlling at least one parameter of the first pulse or the second pulse, for controlling a relation between the first time point and the second time point.

This fourth aspect may generally present the same or corresponding advantages as the former aspects. Effects and features of this fourth aspect are largely analogous to those described above in connection with the first, second and third aspects. Embodiments mentioned in relation to the fourth aspect are largely compatible with the first, second and third aspects.

Thanks to the method, accurate control of selective nerve stimulation may be provided.

The method may be performed by a control unit which is configured to control timing of generation of signals. The controlling of timing of generation of signals may be achieved by the control unit sending a control signal to a stimulation signal generating unit for causing generation of a signal by the stimulation signal generating unit. Thus, the controlling of timing does not relate to actual output of the first and second intermittent current waveforms, respectively, but rather relates to providing a timed control of the stimulation signal generating unit.

According to a fifth aspect, there is provided computer program product comprising computer-readable instructions which when executed by a processing unit cause the processing unit to perform the method according to the fourth aspect.

This fifth aspect may generally present the same or corresponding advantages as the former aspects. Effects and features of this fifth aspect are largely analogous to those described above in connection with the first, second, third and fourth aspects. Embodiments mentioned in relation to the fifth aspect are largely compatible with the first, second, third and fourth aspects.

Thanks to the computer program product, accurate control of selective nerve stimulation may be provided.

The computer program product may thus provide computer-readable instructions for allowing the method to be implemented. This allows the functionality of the method to be provided to any processing unit.

The computer program product may comprise a non-transient computer-readable medium for carrying the computer-readable instructions. Alternatively, in other embodiments, the computer program product may comprise a signal carrying the computer-readable instructions, e.g., for communicating the computer program product to the processing unit through wired or wireless communication.

According to a sixth aspect, there is provided a method for nerve stimulation for pain relief, said method comprising providing a first intermittent current waveform comprising a first pulse to a first pair of electrodes of an electrode arrangement arranged in relation to a sensory nerve at a spinal cord, providing a second intermittent current waveform comprising a second pulse to a second pair of electrodes of the electrode arrangement, stimulating a plurality of neurons in at least a first region and a second region of the sensory nerve using interferential stimulation based on interference between the first intermittent current waveform and the second intermittent current waveform, wherein stimulation of non-noxious sensory neurons is configured to attenuate transmission of pain-related signals by inhibiting relay neurons in the dorsal horn, wherein the interferential stimulation of the neurons is configured to activate stimulation of the neurons in the first region at a first time point, and neurons in the second region at a second time point, and controlling a relation between the first time point and the second time point by the stimulation signal generating unit controlling at least one parameter of the first pulse and the second pulse, wherein the controlling is configured to ensure that the first time point is different from the second time point.

This sixth aspect may generally present the same or corresponding advantages as the former aspects. Effects and features of this sixth aspect are largely analogous to those described above in connection with the first, second, third, fourth and fifth aspects. Embodiments mentioned in relation to the fifth aspect are largely compatible with the first, second, third, fourth and fifth aspects.

Stimulation of a non-noxious sensory neuron may attenuate transmission of pain-related signals to pass through to the brain by inhibiting relay neurons in the dorsal horn. This may also be explained as stimulation of the sensory neurons may attenuate transmission of a pain signals by blocking a downstream relay neuron. It is a realization that attenuation of transmission of pain-related signals, such that the pain-related signals are prevented from propagating to the brain, may be achieved by stimulation of sensory neurons such that downstream relay neurons are attenuated. Thanks to the method, nerve stimulation for pain relief may be provided.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view illustrating a system for controlling selective stimulation of neural tissue of a subject.
Fig. 2a-c schematically illustrates temporal profiles of an interferential signal and expected neuron activation.
Fig. 3a is a schematic view of a system for controlling selective stimulation arranged at a spinal cord and the impact of inferential stimulation focus change on neuron activation across the neural tissue.
Fig. 3b is a schematic view of a system for controlling selective stimulation alternatively arranged at a spinal cord and the impact of inferential stimulation focus change on neuron activation across the neural tissue.
Fig. 4 is a schematical illustration of a method 400 for controlling selective nerve stimulation of neural tissue of a subject.
Fig. 5 is schematical illustration of a computer implemented method for controlling selective nerve stimulation of neural tissue of a subject.
Fig. 6 is a schematical illustration of a method for nerve stimulation for pain relief.

### Detailed description

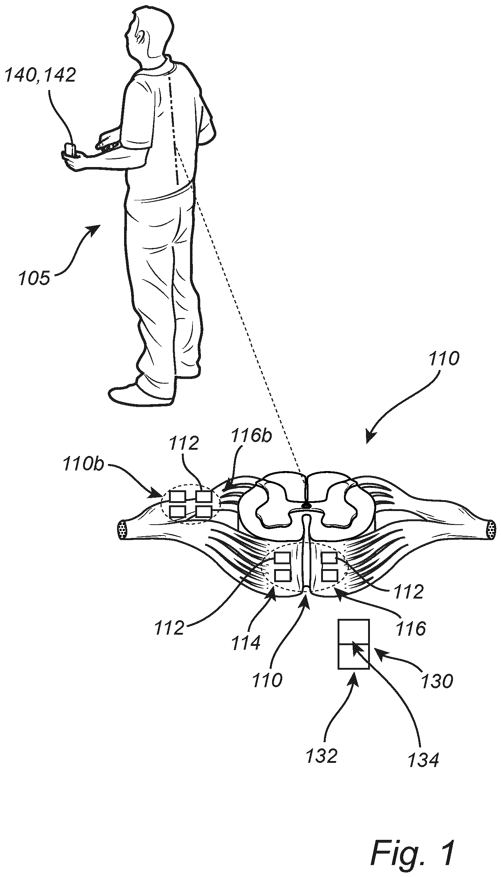

Referring now to Fig. 1, a system 100 for controlling selective stimulation of neural tissue of a subject will be described.

As illustrated in Fig. 1, the system 100 comprises an electrode arrangement 110, a signal generating unit 130 and a sensor arrangement 140.

The electrode arrangement 110 may comprise a plurality of electrodes 112. The electrodes 112 of the plurality of electrodes are preferably arranged in pairs.

In Fig. 1, the electrode arrangement is illustrated having a plurality of electrodes 112, in this case four electrodes, arranged in a first pair of electrodes 114 and a second pair of electrodes 116.

The sensor arrangement 140 may comprise at least one sensor 142. In Fig. 1 the sensor arrangement is illustrated having a single sensor 142, however in another example the sensor arrangement may comprise more than one sensor.

The stimulating signal generating unit 130 may be connected to the electrode arrangement 110. The stimulating signal generating unit 130 may for instance be configured to generate a first intermittent current waveform comprising a first pulse, and further configured to output the first intermittent current waveform to a first pair of electrodes of the electrode arrangement 110, and be configured to generate a second intermittent current waveform comprising a second pulse, and further configured to output the second intermittent current waveform to a second pair of electrodes of the electrode arrangement 110, and thereby causing interferential stimulation of a plurality of neurons in at least a first region and a second region of the neural tissue.

The stimulating signal generating unit 130 may further be connected to a sensor arrangement 140. The sensor arrangement 140 may be configured to detect at least one of a neural activity or a physiological activity of the subject 105, wherein the sensor arrangement is configured to detect indications of changes in at least one of the neural activity or the physiological activity in response to the interferential stimulation of the neurons.

The stimulation signal generating unit may further be configured to control the at least one parameter of the first pulse or the second pulse, based on the indications of changes in at least one of the neural activity or the physiological activity.

The electrode arrangement 110 may be configured to be arranged within a subject 105. In the illustrated example of Fig. 1 the subject 105 is a human being. The electrode arrangement 110 is illustrated arranged at a spinal cord of the subject 105.

As illustrated in Fig. 1, the electrodes 112 of the electrode arrangement 110 may comprise a plurality of pairs of electrodes 112, wherein each pair 114,116 comprises two electrodes displaced longitudinally in relation to each other along a neural tissue, in the illustrated example the spinal cord of the subject 105, at which the electrode arrangement 110 is arranged.

The electrode arrangement 110 may thus be arranged in a first and a second lead, where the first and the second lead comprises at least a pair of electrodes respectively, and wherein the leads are arranged in relation to the spinal cord such that the first and second pair of electrodes are arranged substantially opposite each other. In other words, a first pair electrode 112 of the electrode arrangement is arranged at a first side of the spinal cord, and a second pair electrodes 112 is arranged at an opposite side of the spinal cord.

It should be realized that in other embodiments, other number of electrodes may be used and that a larger or smaller number of electrodes may be used.

Each pair of electrodes form a stimulation location, wherein a stimulation location is established between the first and the second electrode of the pair of electrodes.

The sensor arrangement 140 is illustrated arranged external to the subject 105. The sensor arrangement 140 may comprise a single sensor 142. This sensor 142 is configured to detect indications of changes in a neural activity or a physiological activity in response to the interferential stimulation of the neurons by the electrodes 112. The sensor 142 may be configured to detect indications of change in a single neural activity or a single physiological activity, or may be configured to detect indications of change in more than one neural activity and/or physiological activity.

The sensor arrangement 140 may for example be worn by the subject as a smart watch, smart ring or other external devices. As an alternative, the sensor arrangement 140 may be at least partly arranged within the subject 105.

The stimulation signal comprises properties, for example, a pair of selected electrodes, a current amplitude, a frequency, a duration time of the stimulation, a pulse repetition frequency, and/or a pulse width. These properties of the stimulation signal are related to the detected indications of change in the neural activity and/or physiological activity.

The stimulation signal generator 130 may be configured to generate the first intermittent current waveform and the second intermittent current waveform. The stimulation signal generating unit 130 may comprise separate current generator for generating separate signals. This may imply that the generation of one waveform is not affected by the generation of other waveforms.

For instance, the stimulation signal generating unit 130 may comprise a first current generator 132 configured to generate the first intermittent current waveform, and a second current generator 134 configured to generate the second intermittent current waveform.

The interferential stimulation of each of the neurons of the plurality of neurons in the first region and in the second region may be configured to activate stimulation of the plurality of neurons in a first region at a first time point, and activate stimulation of the plurality of neurons in the second region at a second time point, and thereby initiate propagation of signals within the neural tissue for inhibiting propagation of pain signal within the neural tissue.

A first pair 114 of electrodes 112 of the electrode arrangement 110 are activated, and a second pair 116 of electrodes 112 of the electrode arrangement 110 are activated. The stimulation signal generating unit provides a first intermittent current waveform comprising a first pulse to the first pair of electrodes of an electrode arrangement, and the second intermittent current waveform comprising a second pulse to the second pair of electrodes of the electrode arrangement.

A plurality of neurons in at least a first region and a second region of the neural tissue are stimulated using interferential stimulation based on interference between the first intermittent current waveform and the second intermittent current waveform. The interferential stimulation of the neurons is configured to activate stimulation of the neurons in the first region at a first time point, and neurons in the second region at a second time point, due to difference in amplitude modulation of the interferential signal in the first region and the second region. The relation between the first time point and the second time point is controlled by the stimulation signal generating unit controlling at least one parameter of the first pulse or the second pulse.

The sensor 142 is configured to detect indications of change in a neural or a physiological activity of the subject 105. In a specific example, the sensor 142 may be a pulse monitor. In this example the sensor 142 may detect a indication of change in the heart rate. The stimulation signal generating unit is further configured to control at least one parameter of the first and/or second intermittent current waveform, based on the detected indications of change in the heart rate. This change may for example be the amplitude of the least one of the first pulse and the second pulse. This may result in a different relation between the first time point and the second time point. This feedback-loop may continue until no indications of changes are detected by the sensor, and an optimal stimulation has been achieved.

It is a realization that due to body movements of the subject, the electrodes may change position within the body, and thus cause a change of stimulation locations. This may cause indications of change in the neural and/or physiological activity, and thus cause the system to change at least one parameter of the first and/or second intermittent current waveform.

The system 100 may be used for stimulating a plurality of neurons in at least a first region and a second region of the neural tissue using interferential stimulation, and detecting a neural activity and/or a physiological activity of the subject in response to the interferential stimulation. By detecting the response from the interferential stimulation and controlling a parameter of the first pulse or the second pulse based on the detected response, the system 100 may adjust itself to changes, and create a substantially instant feedback loop.

Fig. 1 further illustrates an alternative placement of an electrode arrangement 110b at a spine of the subject 105. The electrode arrangement 110b may comprise a first pair 114b and a second par 116b of electrodes 112, wherein each pair of electrodes comprises two electrodes displaced longitudinally in relation to each other along a spinal nerve. The spinal nerve may be a spinal ganglion.

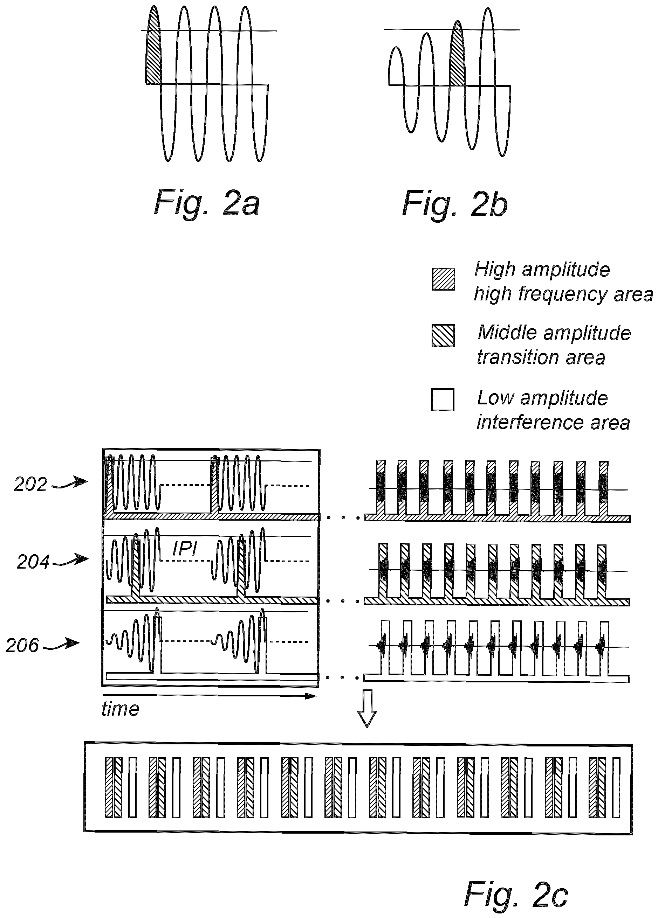

Fig. 2a-b schematically illustrates temporal profiles of an interferential signal. Interaction between the first pulse of the first intermittent current waveform and the second pulse of the second intermittent current waveform, being sinusoidal waves, produces an interferential stimulation pulse having an amplitude modulated waveform.

Two examples of a portion of the interferential stimulation pulse relating to two different regions of the neural tissue is illustrated in Fig. 2a-b. The peak amplitude of cycles is compared to an activation threshold of the neuron.

Fig. 2a illustrates an interferential stimulation pulse subject to substantially no amplitude modulation. As evident from Fig. 2a, the peak amplitude is above the activation threshold for all cycles. Thus, the interferential signal may activate stimulation of a neuron when the first cycle of the interferential signal reaches the activation threshold, such that an action potential is triggered in the neuron. Subsequent cycles will not trigger action potentials due to a refractory period of the neuron, if the subsequent cycles are within said refractory period.

In Fig. 2b, the peak amplitude is below the activation threshold for some cycles before reaching the activation threshold in a cycle. Thus, the interferential signal may activate stimulation of a neuron when a cycle of the interferential signal reaches the activation threshold, such that an action potential is triggered in the neuron. Subsequent cycles will not trigger action potentials due to a refractory period of the neuron, if the subsequent cycles are within said refractory period.

The interferential signal may provide a time delay of fiber activation, which may be different for different neurons. The time delay of activating different neurons may be enhanced by interferential intermittent current stimulation due to the amplitude modulation of the interferential signal, which can result in fiber activation at different cycles within the envelope curve for different neurons.

The interferential stimulation will cause activation of the plurality of neurons within the neural tissue or part of the neural tissue. The activation of the neurons will cause propagation of neural signal through the neurons.

Due to the interferential signal/waveform having an increasing amplitude (i.e., being amplitude modulated) different neurons will be activated at different time points. In addition, the plurality of neurons may have different thresholds for activation further causing different neurons to be activated at different time points. The activation threshold may for example depend on the degree of myelination or the circumference of the neuron.

To further illustrate the temporal difference in activation in different spatial regions of the neural tissue it is now referred to Fig. 2c, providing a schematical illustration of the activation pattern for neurons in different regions of a cross section of the neural tissue.

Depending on the degree of interference between the first intermittent current waveform and the second intermittent current waveform, and thus the degree of amplitude modulation of the stimulation signal, different regions of the neural tissue will experience stimulation activation at different time points.

In a first region which may be arranged close to one of the pairs of electrodes, the interferential signal will experience no to very little amplitude modulation, as indicated by 202, and the peak amplitude is above the activation threshold for all cycles. Hence, the interferential signal will activate stimulation of a neuron, or a plurality of neurons, in the first region when the first cycle of the interferential signal reaches the activation threshold, such that an action potential is triggered in the neuron(s). Subsequent cycles will not trigger action potentials due to a refractory period of the neuron, if the subsequent cycles are within said refractory period. Additionally, the refractory period will prevent other signal, such as pain signals, from propagating further, thereby offering a pain relief.

In a second region which may be arranged at a distance from a surface of the neural tissue and hence farther away from the pairs of electrodes than the first region, the interferential signal will experience more amplitude modulation than in the first region, as indicated by 204. The peak amplitude is below the activation threshold for some cycles before reaching the activation threshold in a cycle. The interferential signal will activate stimulation of a neuron, or a plurality of neurons, in the second region when the third cycle of the interferential signal reaches the activation threshold, such that an action potential is triggered in the neuron(s). Subsequent cycles will not trigger action potentials due to a refractory period of the neuron, if the subsequent cycles are within said refractory period. Additionally, the refractory period will prevent other signal, such as pain signals, from propagating further, thereby offering a pain relief.

The activation in the second region will occur at a second time point, that is different than the first time point.

In a third region which may be arranged deep into the neural tissue, the interferential signal will experience more amplitude modulation than in the second region, as indicated by 206. The peak amplitude is below the activation threshold for all cycles except for the last cycle, where it reaches the activation threshold. The interferential signal will activate stimulation of a neuron, or a plurality of neurons, in the third region when the last cycle of the interferential signal reaches the activation threshold, such that an action potential is triggered in the neuron(s).

The activation in the third region will occur at a third time point, that is different than the first time point and the second time point.

Having a fixed inter-pulse-interval between subsequent pulses will result in a fixed time interval between stimulation activation in different regions, as indicated in Fig. 2c.

Thanks to activation of stimulation of the different regions at different time points, paresthesia may be avoided in case of spinal cord stimulation.

Further, using a fixed time interval between stimulation activation in different regions may ensure that a repetitive stimulation may be provided, and will advantageously avoid causing paresthesia, while also providing a energy efficient stimulation regime.

It may be advantageous to change the repetitive pattern. For example, a posture change of the subject may cause a shift in the relative electrode-neuron position. A first repetitive pattern may be provided for seconds or minutes before it changed to a second repetitive pattern. Alternatively, the repetitive pattern may be changed to avoid, or decrease, neural fatigue and thereby increase the neural activation effectiveness. A first repetitive pattern may be provided for minutes up to hours before it is changed to a second repetitive pattern. It is a realization that depending on where in the subject the electrode arrangement is arranged, it may be affected more or less of the movement artifacts and body posture changes.

The change in the repetitive pattern may be caused by the stimulation signal generating unit controlling at least one parameter of the first and/or the second pulse of the first and the second intermittent current waveform, respectively. The change may be triggered by the sensor arrangement detecting a change in at least one of a neural or a physiological activity.

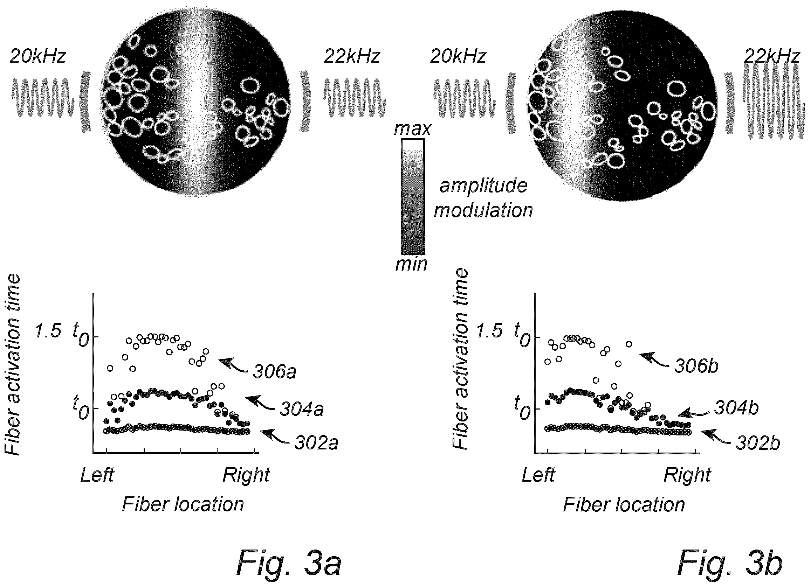

Referring now to Fig. 3a and Fig. 3b, neuron activation time as a function of pulse length and location of the neuron within the neural tissue in relation to the electrodes is illustrated. A cross section of neural tissue is illustrated for reference, having a first pair of electrodes arranged on a left side, and a second pair of electrodes arranged on an opposite, right side of the neural tissue cross section.

Turning to Fig. 3a the left pair of electrodes and the right pair of electrodes provides the same stimulation current. The black circles, 302a, illustrates activation time of the neurons of the neural tissue from left to right using sinusoidal stimulation, such that no difference in stimulation frequency, amplitude and phase between the left pair of electrodes and the right pair of electrodes exists. The sinusoidal stimulation activation time differs very little, or substantially not at all, spatially in the neural tissue.

The crosses, 304a, illustrates activation time of the neurons of the neural tissue from left to right using interferential stimulation with shorter pulses. The shorter pulses provide a more distinct difference in neuron activation time depending on the neuron's spatial location in relation to the electrodes. Neurons arranged deep into the neural tissue have an increased activation time delay, compared to neurons arranged close to one of the pairs of electrodes. In other words, neurons located close to the electrodes will be activated before neurons located closer to the middle of the neural tissue, when the neural tissue is observed as a cross section. Having the right pair of electrodes provide the same current amplitude as the left pair of electrodes, the neurons arranged at the same distance from a pair of electrodes, independently of if it is the right pair of electrodes or the left pair of electrodes, will be activated at substantially the same time. In other words, when both pairs of electrodes provide the same current amplitude, the neurons arrangement in relation to the pairs of electrodes may be a primary reason for an activation time delay.

The circles, 306a, illustrates activation time of the neurons of the neural tissue from left to right using longer pulses. A longer pulse will further increase the activation time delay between neurons arranged deep in the neural tissue and neurons arranged closer to the electrodes, due to the even slower ramp-up of the stimulus at the point of interference. In other words, there will be a larger difference in time between the activation time of neurons closer to the pairs of electrodes, compared to neuron arranged deeper in the neural tissue.

The cross section of neural tissue illustrated for reference shows a first pair of electrodes arranged on a left side, providing a first pulse having a first frequency, and a second pair of electrodes arranged on an opposite, right side of the neural tissue cross section, providing a second pulse having a second frequency. In this example, the first frequency is 20 kHz and the second frequency is 22 kHz. Further, the current amplitude of the first pulse is the same as the current amplitude of the second pulse.

The cross section further illustrates the difference in the degree of amplitude modulation throughout the cross section of the neural tissue. The degree of interference between the first pulse and the second pulse, and thus the degree of amplitude modulation of the stimulation signal, vary in different regions of the neural tissue.

As illustrated, a region arranged deep into the neural tissue experience a stimulation signal having a high degree of amplitude modulation and neurons arranged deep into the neural tissue will thus have an increased activation time delay.

A region arranged close to one of the pairs of electrodes experience a stimulation signal having a lower degree of amplitude modulation and neurons arranged close to one of the pairs of electrodes will thus have a decreased activation time delay, compared to the neurons arranged deep into the neural tissue.

Turning to Fig. 3b, the right pair of electrodes provides a higher stimulation current compared to the left pair of electrodes. Having one pair of electrodes provide a lower or a higher current compared to the other pair of electrodes will cause a temporal and spatial displacement of the neuron activation time.

The black circles, 302b, illustrates activation time of the neurons of the neural tissue using sinusoidal stimulation, such that no difference in stimulation frequency and phase between the left pair of electrodes and the right pair of electrodes exists, except the amplitude difference. With the sinusoidal stimulation neuron activation time differs very little, or substantially not at all, spatially in the neural tissue.

The crosses, 304b, illustrates activation time of the neurons of the neural tissue from left to right using shorter pulses. The shorter pulses provide a more distinct difference in neuron activation time depending on the neuron's spatial location in relation to the electrodes. Neurons arranged deep into the neural tissue have an increased activation time delay, compared to neurons arranged close to one of the pairs of electrodes.

Furthermore, the right pair of electrodes providing a higher current than the left pair of electrodes moves the focus of interference on the left side of the nerve, resulting in temporal activation delay for neurons located on the left side, wherein the neurons arranged closer to the right pair of electrodes will be activated before the neurons arranged closer to the left pair of electrodes, as they experience lower stimulus amplitude modulation.

Depending on the current provided by the right and left pair of electrodes, respectively, it is possible to accurately control the activation time depending on where the neuron is arranged within the neural tissue.

The circles, 306b, illustrates activation time of the neurons of the neural tissue from left to right using longer pulses. As discussed in relation to Fig. 3a, a longer pulse will further increase the activation time delay between neurons arranged deep in the neural tissue and neurons arranged closer to the electrodes. Furthermore, the right pair of electrodes providing a higher current than the left pair of electrodes will cause the neurons arranged closer to the right pair of electrodes to be activated before the neurons arranged closer to the left pair of electrodes. This essentially provides a shift in which region has the latest activation time.

The cross section of neural tissue illustrated for reference, shows a first pair of electrodes arranged on a left side, providing a first pulse having a first frequency, and a second pair of electrodes arranged on an opposite, right side of the neural tissue cross section, providing a second pulse having a second frequency. In this example, the first frequency is 20 kHz and the second frequency is 22 kHz. Further, the current amplitude of the second pulse is approximately two times the current amplitude of the first pulse.

The cross section further illustrates the difference in the degree of amplitude modulation throughout the cross section of the neural tissue. The degree of interference between the first pulse and the second pulse, and thus the degree of amplitude modulation of the stimulation signal, vary in different regions of the neural tissue. As mentioned, one pair of electrodes provides a lower or a higher current compared to the other pair of electrodes. This causes a spatial displacement of the region experiencing the stimulation signal having the highest degree of amplitude modulation. By having the pair of electrodes arranged at the right side of the neural tissue cross section provide a higher current amplitude, a spatial displacement to the left within the cross section will occur.

As illustrated, a region arranged closer to the electrode pair providing the lower current amplitude than the electrode pair providing the higher current amplitude experiences a stimulation signal having a high degree of amplitude modulation and neurons arranged in this region of the neural tissue will thus have an increased activation time delay. A region arranged closer to the pair of electrodes providing the higher current amplitude experiences a stimulation signal having a lower degree of amplitude modulation and neurons arranged close to the pair of electrodes providing the higher current amplitude will thus have a decreased activation time delay, compared to the neurons arranged deep into the neural tissue.

Fig. 3a and 3b effectively illustrates the relation between a first time point and a second time point based on control of the pulse length and/or the current amplitude of the first pulse and/or the second pulse.

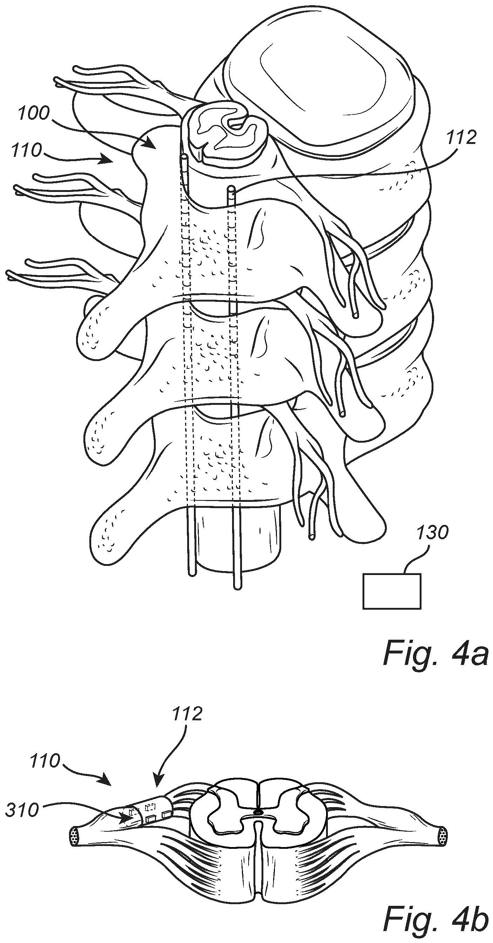

Referring now to Fig. 4a, the system is illustrated arranged at a spine of a subject.

The system 100 comprises electrode arrangement 110 and a signal generating unit 130. The electrode arrangement 110 comprises a plurality of electrodes.

In Fig. 3a, the electrode arrangement 110 is illustrated comprising 112 electrodes. The plurality of electrodes 112 are arranged in a fist and a second wire and may be configured to be inserted in the spine between the spinal cord and the epidural space. The wires, and thus the electrodes 112, are brought into contact with the spinal cord.

Referring now to Fig. 4b, a schematical illustration of an alternative arrangement of the system at a spine of a subject.

As illustrated in Fig. 4b the electrodes 112 of the electrode arrangement 110 may comprise a plurality of pairs of electrodes, wherein each pair of electrodes comprises two electrodes displaced longitudinally in relation to each other along a spinal nerve. The spinal nerve may be a spinal ganglion. The electrode arrangement may thus be arranged in a carrier 310 formed as a cuff, which is arranged around the spinal nerve for placing the electrodes 112 in relation to the spinal nerve.

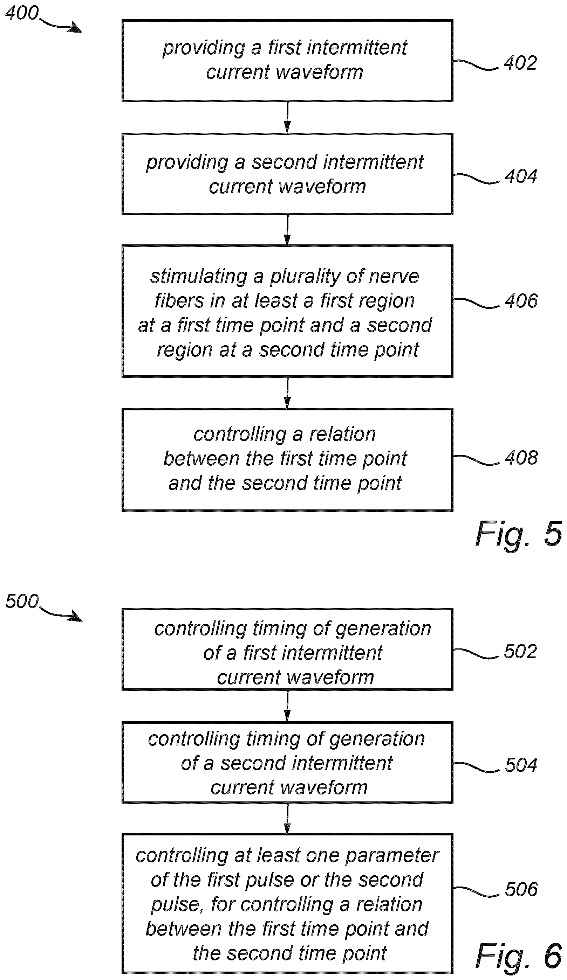

Referring now to Fig. 5, a schematical illustration of a method 400 for controlling selective nerve stimulation of neural tissue of a subject is shown.

As indicated by block 402, the method 400 comprises providing a first intermittent current waveform comprising a first pulse to a first pair of electrodes of an electrode arrangement, and, as indicated by block 404, providing a second intermittent current waveform comprising a second pulse to a second pair of electrodes of the electrode arrangement.

It is a realization that providing 402 the first intermittent current waveform and providing 404 the second intermittent current waveform may occur substantially simultaneously.

Thereafter, as indicated by block 406, the method 400 further comprises stimulating a plurality of neurons in at least a first region and a second region of the neural tissue using interferential stimulation based on interference between the first intermittent current waveform and the second intermittent current waveform. The interferential stimulation of the neurons is configured to activate stimulation of the neurons in the first region at a first time point, and neurons in the second region at a second time point.

Lastly, the method 400 comprises controlling a relation between the first time point and the second time point by the stimulation signal generating unit controlling at least one parameter of the first pulse or the second pulse, as indicated by block 408.

Referring now to Fig. 6, a schematical illustration of a computer implemented method 500 for controlling selective nerve stimulation of neural tissue of a subject is shown.

As indicated by block 502, the method 500 comprises controlling timing of generation of a first intermittent current waveform comprising a first pulse to be output to a first pair of electrodes of an electrode arrangement, and, as indicated by block 504, controlling timing of generation of a second intermittent current waveform comprising a second pulse to be output to a second pair of electrodes of the electrode arrangement. Timing of the first and second intermittent current waveform controls timing of stimulation of a plurality of neurons in at least a first region and a second region of the neural tissue using interferential stimulation based on interference between the first intermittent current waveform and the second intermittent current waveform.

The interferential stimulation of each of the neurons of the plurality of neurons in a first region is configured to activate stimulation of the neuron at a first time point, and the interferential stimulation of each of the neurons of the plurality of neurons in a second region is configured to activate stimulation of the neuron at a second time point.

As indicated by block 506, the method 500 further comprises controlling at least one parameter of the first pulse or the second pulse, for controlling a relation between the first time point and the second time point.

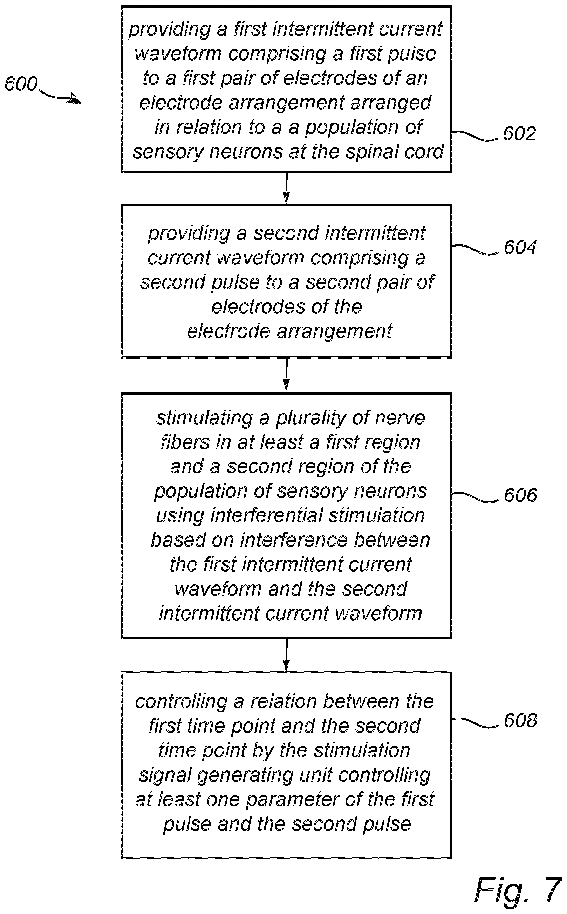

Referring now to Fig. 7, a schematical illustration of a method 600 for nerve stimulation for pain relief is shown.

As indicated by block 602, the method 600 comprises providing a first intermittent current waveform comprising a first pulse to a first pair of electrodes of an electrode arrangement arranged in relation to a sensory nerve or a population of neurons at a spinal cord, and, as indicated by block 604 providing a second intermittent current waveform comprising a second pulse to a second pair of electrodes of the electrode arrangement.

Thereafter, the method 600 comprises stimulating a plurality of neurons in at least a first region and a second region of the sensory neurons using interferential stimulation based on interference between the first intermittent current waveform and the second intermittent current waveform, as indicated by block 606. The stimulation of non-noxious sensory neurons is configured to attenuate transmission of pain-related signals by inhibiting relay neurons in a dorsal horn,, and the interferential stimulation of the neurons is configured to activate stimulation of the neurons in the first region at a first time point, and neurons in the second region at a second time point.

Lastly, as indicated by block 608, the method 600 comprises controlling a relation between the first time point and the second time point by the stimulation signal generating unit controlling at least one parameter of the first pulse and the second pulse, wherein the controlling is configured to ensure that the first time point is different from the second time point.

The sensation of pain may start in receptor nerve cells, also called nociceptive nerves, which may be found beneath the skin and in organs throughout the body. If the nociceptors detect damage or potential harm, the nociceptors transmit pain signals through nerve pathways to the spinal cord and then further to the brain.

In chronic pain, the normal pain signaling pathways may become dysregulated. The dysregulation may include an increased activity of calcium channels, which may lead to an excessive release of neurotransmitters and persistent pain signals.

On the other hand, stimulation of non-noxious sensory neurons attenuates transmission of pain-related signals to pass through to the brain by inhibiting relay neurons in the dorsal horn.

By stimulating the spine, a majority, or substantially all, pain signals may be blocked from propagating, and thereby preventing the subject's sensation of pain. Attenuation of transmission of signals may be sufficiently strong such that the pain signal is completely blocked and/or prevented. Blocking of a signal should thus be understood as a case of strong attenuation. The attenuation may be achieved at downstream relay neurons, wherein the downstream relay neurons have a common input from both sensory neurons and pain neurons. Hence, sensory neurons may be stimulated to attenuate signal from pain neurons.

To achieve pain relief for chronic pain the method 600 for nerve stimulation for pain relief may be used continuously over a long period of time, for example months or years.

To avoid neural fatigue and adapt to movement artifacts, the system advantageously adapts itself by controlling the relation between the first time point and the second time point by the stimulation signal generating unit controlling at least one parameter of the first pulse and the second pulse, and thereby control the a repetitive activation pattern provided by a sequence of pulses.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system (100) for controlling selective stimulation of neural tissue of a subject, said system comprising:
an electrode arrangement (110), comprising a plurality of electrodes (112), wherein different electrodes of the plurality of electrodes are configured to be arranged in different electrode locations in relation to the neural tissue, and
a stimulation signal generating unit (130),
wherein the stimulation signal generating unit is configured to generate a first intermittent current waveform comprising a first pulse and a second intermittent current waveform comprising a second pulse, wherein the first pulse comprises a first frequency and the second pulse comprises a second frequency, wherein the second frequency is different from the first frequency,
wherein the stimulation signal generating unit is configured to output the first intermittent current waveform to a first pair of electrodes of the electrode arrangement and the second intermittent current waveform to a second pair of electrodes of the electrode arrangement, for causing interferential stimulation of a plurality of neurons in at least a first region (122) and a second region (124) of the neural tissue,
wherein the interferential stimulation of the neurons is configured to activate stimulation of the neurons in the first region at a first time point, and the neurons in the second region at a second time point,
wherein the stimulation signal generating unit is configured to control at least one parameter of the first pulse or the second pulse, for controlling a relation between the first time point and the second time point.

2. The system according to claim 1, further comprising
a sensor arrangement configured to detect at least one of a neural activity or a physiological activity of the subject, wherein the sensor arrangement is configured to detect indications of changes in at least one of the neural activity or the physiological activity in response to the interferential stimulation of the neurons, and wherein the stimulation signal generating unit is configured to control the at least one parameter of the first pulse or the second pulse, based on the indications of changes in at least one of the neural activity or the physiological activity.

3. The system according to claim 2, further comprising:
a control unit configured to determine settings of the stimulation signal generating unit, wherein the control unit is configured to:
store a pattern representing a desired time relation between the first time point and the second time point,
receive feedback from the sensor arrangement representing an actual time relation between nerve signals relating to neurons in the first region and neurons in the second region,
compare the received feedback to the stored pattern for determining a necessary change in the settings of the stimulation signal generating unit; and
send a control signal to the stimulation signal generating unit for causing the stimulation signal generating unit to change that at least one parameter of the first pulse or the second pulse.

4. The system according to any one of the preceding claims, wherein the at least one parameter of the first pulse or the second pulse, comprises at least one of
an amplitude,
a frequency,
a starting time,
a starting phase, or
a pulse length.

5. The system according to any one of the preceding claims, wherein the first intermittent current waveform comprises a first sequence of pulses and the second intermittent current waveform comprises a second sequence of pulses, and wherein the stimulation signal generating unit is configured to control an amplitude of a pulse of the first sequence following a preceding pulse of the first sequence, and control an amplitude of a pulse of the second sequence following a preceding pulse of the second sequence.

6. The system according to claim 5, wherein the simulation signal generating unit is further configured to control the inter-pulse-interval in the first sequence of pulses and the second sequence of pulses.

7. The system according to any one of the preceding claims, wherein the first region and the second region are different spatial regions in a cross-section of the neural tissue.

8. The system according to any one of the preceding claims, wherein at least one of an envelope maximum or an envelope minimum of the interferential stimulation is different in the first region and in the second region.

9. The system according to any one of the preceding claims, wherein the stimulation signal generating unit comprises
a first current generator, configured to generate the first intermittent current waveform, and
a second current generator, configured to generate the second intermittent current waveform.

10. The system according to any one of the preceding claims, wherein the system is configured to be at least partially implanted at a spine of the subject.

11. A method for controlling selective nerve stimulation of neural tissue of a subject, said method comprising:
providing a first intermittent current waveform comprising a first pulse to a first pair of electrodes of an electrode arrangement,
providing a second intermittent current waveform comprising a second pulse to a second pair of electrodes of the electrode arrangement,
stimulating a plurality of neurons in at least a first region and a second region of the neural tissue using interferential stimulation based on interference between the first intermittent current waveform and the second intermittent current waveform, wherein the interferential stimulation of the neurons is configured to activate stimulation of the neurons in the first region at a first time point, and neurons in the second region at a second time point; and
controlling a relation between the first time point and the second time point by the stimulation signal generating unit controlling at least one parameter of the first pulse or the second pulse.

12. A device for controlling selective nerve stimulation of neural tissue of a subject, said device being configured to:
control timing of generation of a first intermittent current waveform comprising a first pulse to be output to a first pair of electrodes of an electrode arrangement,
control timing of generation of a second intermittent current waveform comprising a second pulse to be output to a second pair of electrodes of the electrode arrangement,
wherein timing of the first and second intermittent current waveform controls timing of stimulation of a plurality of neurons in at least a first region and a second region of the neural tissue using interferential stimulation based on interference between the first intermittent current waveform and the second intermittent current waveform, and wherein the interferential stimulation of each of the neurons of the plurality of neurons in a first region is configured to activate stimulation of the neuron at a first time point, and the interferential stimulation of each of the neurons of the plurality of neurons in a second region is configured to activate stimulation of the neuron at a second time point, and
wherein the device is further configured to control at least one parameter of the first pulse or the second pulse, for controlling a relation between the first time point and the second time point.

13. A computer implemented method for controlling selective nerve stimulation of neural tissue of a subject, said method comprising:
controlling timing of generation of a first intermittent current waveform comprising a first pulse to be output to a first pair of electrodes of an electrode arrangement,
controlling timing of generation of a second intermittent current waveform comprising a second pulse to be output to a second pair of electrodes of the electrode arrangement, wherein timing of the first and second intermittent current waveform controls timing of stimulation of a plurality of neurons in at least a first region and a second region of the neural tissue using interferential stimulation based on interference between the first intermittent current waveform and the second intermittent current waveform, wherein the interferential stimulation of each of the neurons of the plurality of neurons in a first region is configured to activate stimulation of the neurons at a first time point, and the interferential stimulation of each of the neuron of the plurality of neurons in a second region is configured to activate stimulation of the neuron at a second time point, and
controlling at least one parameter of the first pulse or the second pulse, for controlling a relation between the first time point and the second time point.

14. A computer program product comprising computer-readable instructions which when executed by a processing unit cause the processing unit to perform the method according to claim 14.

15. A method for nerve stimulation for pain relief, said method comprising:
providing a first intermittent current waveform comprising a first pulse to a first pair of electrodes of an electrode arrangement arranged in relation to a population of sensory neurons at a spinal cord,
providing a second intermittent current waveform comprising a second pulse to a second pair of electrodes of the electrode arrangement,
stimulating a plurality of neurons in at least a first region and a second region of the population of sensory neurons using interferential stimulation based on interference between the first intermittent current waveform and the second intermittent current waveform, wherein stimulation of non-noxious sensory neurons is configured to attenuate transmission of pain-related signals by inhibiting relay neurons in a dorsal horn, wherein the interferential stimulation of the neurons is configured to activate stimulation of the neurons in the first region at a first time point, and neurons in the second region at a second time point; and
controlling a relation between the first time point and the second time point by the stimulation signal generating unit controlling at least one parameter of the first pulse and the second pulse, wherein the controlling is configured to ensure that the first time point is different from the second time point.
